# EUROPEAN PATENT SPECIFICATION

(11) **EP 3 278 131 B1**
(45) Date of publication and mention of the grant of the patent: **06.10.2021**
(21) Application number: 16719930.6
(22) Date of filing: 30.03.2016
(51) Int. Cl.: G01R 33/44, G01R 33/38

(54) **SYSTEM FOR NON-INVASIVE REAL TIME MAGNETIC RESONANCE ANALYSIS OF BODY TISSUE**
SYSTEM ZUR NICHTINVASIVEN MAGNETRESONANZUNTERSUCHUNG VON KÖRPERGEWEBE IN ECHTZEIT
SYSTÈME POUR ANALYSE PAR RÉSONANCE MAGNÉTIQUE NON INVASIVE EN TEMPS RÉEL DE TISSUS CORPORELS

(30) Priority: 30.03.2015 IL 23803415
(43) Date of publication of application: 07.02.2018
(73) Proprietor: Shmri Haim Ltd., 9546507 Jerusalem (IL)
(72) Inventor: LEVY, Yinon, 9546507 Jerusalem (IL)
(74) Representative: Dall'Olio, Christian
(86) International application number: PCT/IL2016/050337
(87) International publication number: WO 2016/157182

(56) References cited:
- JP-A- S62 106 756
- US-A- 5 390 673
- US-A1- 2005 021 019
- US-A1- 2005 040 823
- US-A1- 2013 127 466
- BLUMICH B ET AL: "Mobile single-sided NMR", PROGRESS IN NUCLEAR MAGNETIC RESONANCE SPECTROSCOPY, PERGAMON PRESS, OXFORD, GB, vol. 52, no. 4, 1 May 2008 (2008-05-01), pages 197-269, XP022589395, ISSN: 0079-6565, DOI: 10.1016/J.PNMRS.2007.10.002 [retrieved on 2007-12-28]

## Description

### FIELD OF THE INVENTION

This invention relates to analysis and detection of body tissue using nuclear magnetic resonance (NMR).

### BACKGROUND OF THE INVENTION

Nuclear magnetic resonance is a non-invasive method which is well known for its use in medical imaging (MRI). Its effectiveness for radiological diagnosis is a result of the ability to differentiate between different types of tissues based on the different responses of the water content of the tissue to sequences of RF pulses. Specifically the contrasts in MR images are a result of different relaxation times of the tissue after RF excitation known as T1 and T2.

US 2015/0018638 provides techniques for non-invasive measurement of blood related parameters based on NMR (nuclei) relaxation techniques carried out using a relatively low constant magnetic field in the range of 0.15 to 0.5 Tesla. A plurality of electromagnetic excitation pulse sequences having relatively low radiofrequencies are applied over a living tissue placed in the magnetic field and blood related parameters of the examined subject are determined using a plurality of nuclear spin echo signals received from the tissue in response to the applied excitation sequences, thereby allowing to improve the accuracy of the obtained signals and substantially reducing the time duration of the process.

The apparatus described in US 2015/0018638 is in essence a reduced-profile NMR system that is configured for measuring only a single limb of a patient rather than the whole body and requires insertion of the limb into a receptacle surrounded by a pair of permanent magnets.

MarginProbe™ manufactured and patented by Dune Medical Devices Ltd. of Caesarea, Israel discloses a disposable probe unit housing a proprietary Fringe Field Sensor (FFS). The surgeon applies the probe to the specimen and the FFS senses minute differences in bioelectric properties, enabling it to accurately capture the tissue's electromagnetic signature (healthy or cancerous). The technology behind the MarginProbe System is based on the principle of RF spectroscopy whereby tissue is subjected to an electric field, allowing measurement of the tissue response to that field, yielding an electromagnetic "signature." Positioned at the tip of the probe, the sensor both emits the precisely controlled electromagnetic field and captures the tissue response.

US 2005/0021019 A1, against which claim 1 has been delimited, discloses an apparatus and an method for examining a substance volume to characterize its type, especially for characterizing it as cancerous or non-cancerous. In particular, one applies a polarizing magnetic field and applies RF pulses locally to the examined substance volume such as to invoke magnetic resonance signals corresponding to the MR properties of the substance. Finally, one uses the MR response signal for characterizing the examining substance volume type.

US 2007/222433 discloses a sensor array mounted on a probe body having a distal portion which can be inserted through a minimally invasive aperture for NMR mapping of body tissue.

Reference is also made to an article entitled *"*NMR Properties of Human Median Nerve at 3 T:Proton Density, T1, T2, and Magnetization Transfer" in JOURNAL OF MAGNETIC RESONANCE IMAGING 29:982-986 (2009) by Giulio Gambarota *et al.* This article discusses the measurement of MRI-relevant properties, such as proton density (PD), T1 and T2 relaxation times, and magnetization transfer (MT) in human median nerve at 3 T in order to distinguish between nerve and muscle tissue. The authors conclude that discrimination between median nerve and muscle tissue is difficult.

There is a need for a portable probe that allows real-time determination of tissue type during surgery. Such a probe would allow discrimination between body tissue such as muscle, sinews and the like on the one hand, which can safely be cut during surgery and will heal thereafter and nerves, on the other hand, which should not be damaged during surgery and whose inadvertent damage may be permanent. This need has not been addressed still less met by the prior art.

### SUMMARY OF THE INVENTION

It is therefore an object of the invention to provide a portable probe that allows real-time determination of tissue type during surgery.

This object is realized in accordance with the invention by a system according to claim 1. Preferred embodiments are set out in the dependent claims.

The system according to the invention allows differentiation between nerve tissue and other tissue types such as muscle etc. based on recording and analyzing the T1 and T2 relaxation curves. To this end, the system includes a probe comprising a permanent magnet and an RF coil creating a magnetic field in the range of 0.05 Tesla - 0.5 Tesla at the surface of the probe. The probe is placed on the tissue and full T1 and T2 relaxation curves are recorded as well as magnetization transfer coefficients. The relaxation curve data enables differentiation between the muscle and nerve in real time, which is not possible in an image. Specifically, the relaxation curves are best fit using statistical processing to appropriate single and multi-exponential functions for T1 and T2 respectively. The resulting time constants and weightings for the different exponents are then analyzed based on an ever-growing database accumulated with on-going usage and state of the art clustering algorithms.

### BRIEF DESCRIPTION OF THE DRAWINGS

In order to understand the invention and to see how it may be carried out in practice, embodiments will now be described, by way of non-limiting example only, with reference to the accompanying drawings, in which:
**Fig. 1** is a block diagram showing the functionality of a system according to the invention;
**Figs. 2a, 2b and 2c** are graphical representations useful in explaining operation of the system;
**Fig. 3** is a pictorial representation of a probe for use in the system of Fig. 1;
**Fig. 4** shows pictorially a detail of the magnetic source unit of the probe shown in Fig. 2; and
**Figs. 5a to 5f** are schematic representations showing magnetic source units having different geometries.

### DETAILED DESCRIPTION OF EMBODIMENTS

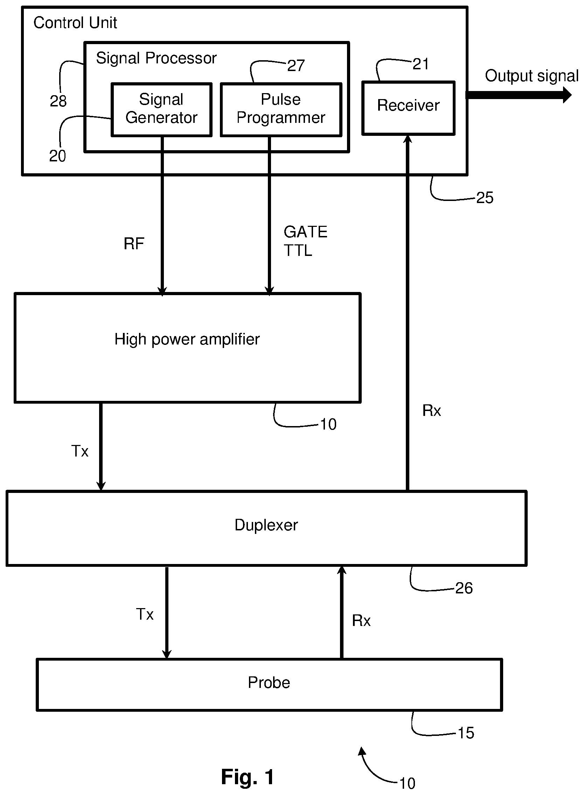

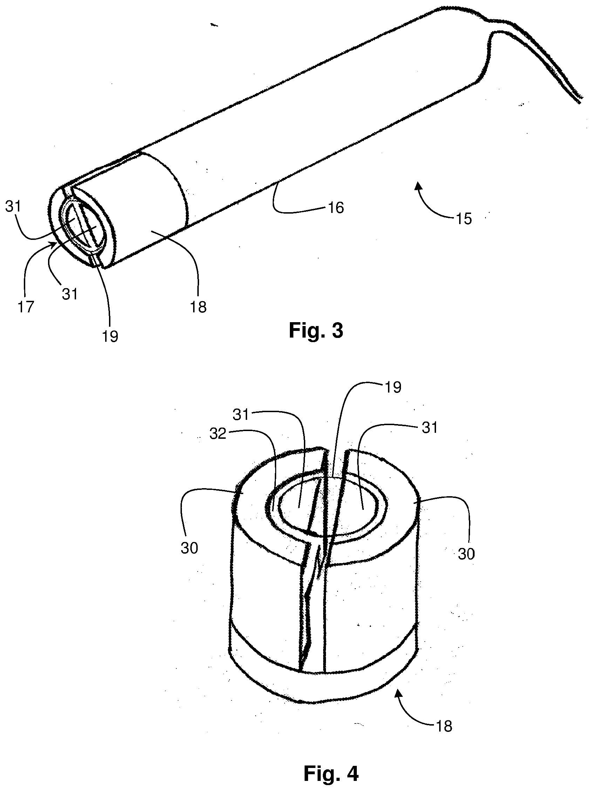

Fig. 1 is a block diagram showing the functionality of a system 10 according to the invention for non-invasive analysis of tissue of an examined subject in order to distinguish between different types of tissue. The system 10 comprises a hand-held probe 15 shown pictorially in Figs. 3 and 4 having a casing 16 formed of non-ferromagnetic material and having a predetermined cross-section at a working end 17 thereof. A magnetic field source unit 18 within the casing is configured to generate a substantially uniform, time-invariant magnetic field within a volume of tissue having a cross-section equal to that of the casing at the working end 17 and having a magnetic field strength in a range of 0.05 to 0.5 Tesla. Disposed within the magnetic field source unit 18 is at least one inductive coil 19 configured to receive RF excitation signals in a specific frequency range typically in the range of 2-20 MHz, the excitation frequency being related to the magnetic field strength such that for each Tesla of magnetic field strength the RF excitation signal is 42 MHz. The inductive coil responds to the magnetic field and to the RF excitation signals by generation of electromagnetic excitation signals in a direction substantially perpendicular to a direction of the magnetic field to thereby magnetize a slice of the living tissue. The slice has a depth and a thickness that are a predetermined function of the magnetic field strength whereby only living tissue in the thus excited slice generates an electromagnetic response to nuclear spin echo signals.

A signal generator 20 is coupled to the inductive coil 19 and is configured for generating an RF excitation frequency that is a function of the respective electromagnetic response of the slice of tissue excited by the probe 10. Likewise, a receiver unit 21 is coupled to the inductive coil 19 and is configured to receive therefrom the electromagnetic response and generate measured data indicative thereof. A control unit 25 is connected to or contains the signal generator 20 so as to generate predetermined time patterns of the excitation RF signals, the control unit 25 being further connected to the receiver unit 21 and responsive to the electromagnetic response for processing the measured data and extracting data indicative of the nuclear spin echo signals from the living tissue, to determine relaxation times and determine therefrom the type of tissue excited by the probe.

In some embodiments, the inductive coil 19 is commonly coupled to the signal generator 20 and to the receiver unit 21 via a duplexer 26 so that the at least one inductive coil applies the RF excitation frequency and receives the response in different time slots.

In some embodiments, the cross-section at the working end 17 of the casing 16 is cylindrical having a diameter of 2-30 mm. In a prototype reduced to practice, the diameter of casing 16 at its working end 17 was 20 mm and permitted analysis of tissue to a depth of between 3.2-9.5 mm at a magnetic field strength B0 of 42-62 mT.

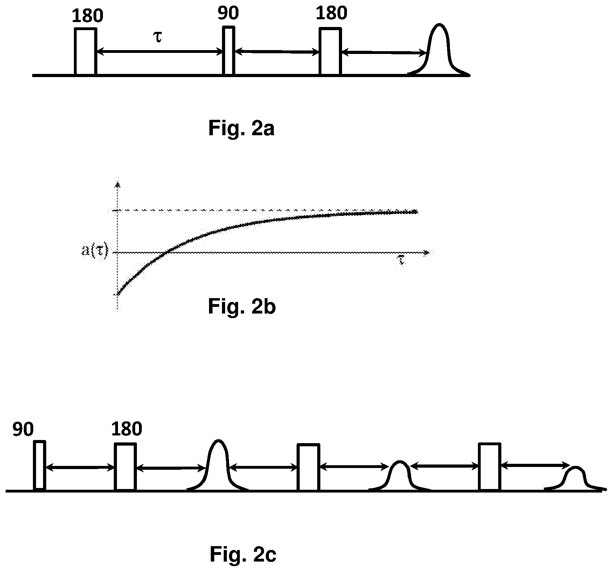

In order to excite and receive response signals from multiple slices at successive depths of body tissue, two different approaches may be employed that may be complementary or used in combination. Both approaches rely on generating and applying via the probe 15 signals of different frequencies, each adapted to magnetize a different slice of the tissue whose depth and a thickness are a predetermined function of the magnetic field strength. According to the invention, there are provided more than one inductive coil each configured to receive a respective RF excitation signal in mutually different frequency ranges so that different slices of tissue are excited simultaneously. In another approach, outside the scope of the invention, the signal generator 20 generates time-varying excitation signals that are applied successively to the same induction coil. Likewise, although in Fig. 1 the same induction coil is used to both apply the excitation signal and receive the response signal, the duplexer 26 serving to direct the signal flow appropriately, multiple induction coils may alternatively be used: one to transmit and one to receive thus obviating the need for the duplexer 26. In either approach, the control unit 25 includes a pulse programmer 27 coupled to the signal generator 20 for obtaining the correct time-varying excitation signals as described below with reference to Figs. 2a to 2c of the drawings. It should be noted that according to the invention more than one inductive coil is provided for simultaneously exciting multiple slices at successive depths of body tissue in mutually different frequency ranges. The signal generator 20 and the pulse programmer 27 together constitute a signal processor 28.

Figs. 3 and 4 show an embodiment of the magnetic field source unit 18 which comprises a pair of outer arcuate segments 30 both of a first magnetic polarity and a pair of inner segments 31 both of a second magnetic polarity opposite to the first magnetic polarity defining an annular gap 32. At least one inductive coil 19 is disposed within the annular gap 32 between the outer and inner segments. The outer and inner segments 30, 31 have respective contours that lie on circles of different radii, the radius of the outer segments being preferably less than 10 mm. The inner segments 31 may be shorter in height than the outer segments 30, in which case they may be supported so that respective end faces of the inner and outer segments are co-planar. When multiple coils are provided, they may be arranged in a vertical stack with the annular gap 32 between the inner and outer segments.

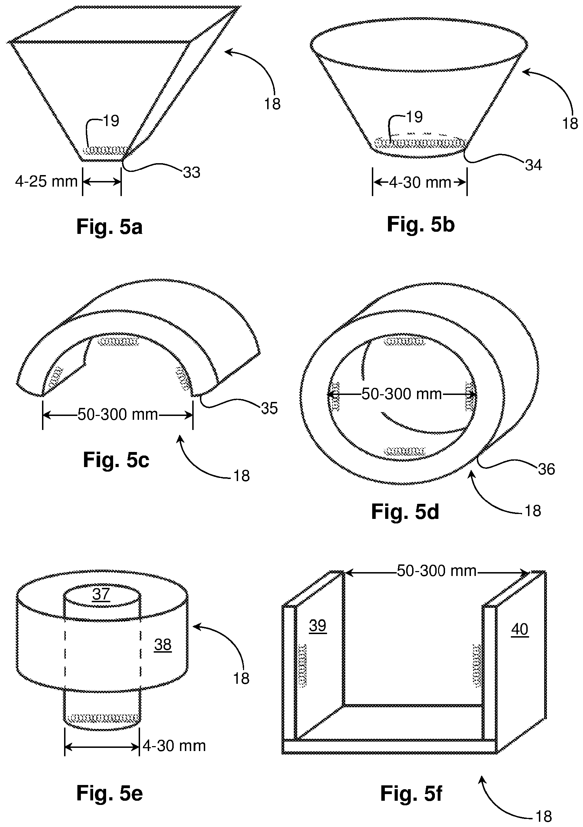

Figs. 5a to 5f are schematic representations showing magnetic source units 18 having different geometries. In all cases, one or more coils are mounted at a working end of the magnetic source unit 18, which is held against the patient's tissue or, in some cases, into which the patient's tissue is inserted. Thus, in Fig. 5a there is shown a magnetic source unit 18 having a generally pyramidal shape with an apex 33 between 4-25 mm and a coil 19 disposed at the apex 33. Fig. 5b shows a magnetic source unit 18 having a conical shape with a coil 19 disposed at a truncated tip 34 of diameter between 4-30 mm. Either of these magnetic source units 18 replaces the head of the probe shown in Fig. 3. Fig. 5c shows a magnetic source unit 18 having the general shape of a banana 35 in the middle of which and at opposite ends of which are provided respective coils 19. Tip-to-tip dimensions are between 50-300 mm. Fig. 5d shows a magnetic source unit 18 having the general shape of a hollow bagel 36 along whose internal surfaces are provided respective coils 19, four such coils being shown each at opposite ends of mutually perpendicular inner diameters of the bagel of dimensions between 50-300 mm. Fig. 5e shows a magnetic source unit 18 having inner and outer cylinders 37 and 38, respectively, a coil 19 being mounted at an end of the inner cylinder 37 of diameter between 4-30 mm. Fig. 5f shows a magnetic source unit 18 having opposing side walls 39 and 40 spaced apart between 50-300 mm, each of which supports coils 19 and between which a patient's limb may be inserted. In all cases, a single coil 19 is shown for clarity, it being understood that in practice multiple coils can be employed.

The system 10 may employ multiple probes 15 each directed for exciting a different portion of body tissue.

Having described the elements of the system 10 and the probe 15, we will now briefly describe the manner in which measurement and analysis are carried out by the control unit 25 with particular reference to Figs 2a to 2c.

In an embodiment of the invention the control unit 25 is configured in real time to access predetermined data characterizing multiple groups of relaxation curves each group defining at least two characteristic curves corresponding to a specific known tissue type and decaying exponentially at respective predetermined time constants T1 and T2 and to analyze measured data in real time to determine to which group of relaxation curves the measured data is best fit in order to identify the tissue type corresponding to the measured data.

The derivation of the time constants T1 and T2 is described below it being understood that the characteristic curves have different time constants T1 and T2 that are characteristic of a specific type of body tissue. The relaxation curves for each different type of body tissue are determined off-line and data representative of the time constants T1 and T2 are stored in a memory of the control unit 25 and statistically processed. During actual measurement, the response signals returned by each slice of tissue to the probe are best fit to an appropriate exponential function resulting in an estimate of the time constant, whereupon it can be established which tissue type corresponds to the time constant obtained by the fit for the slice being measured.

It should be noted that the simultaneous matching of two or more relaxation curves each having predetermined time constants that are stored in the memory of the control unit 25 speeds up the convergence of the best-fit process. For this reason there are many surgical applications where it is essential to use two or more relaxation curves. In many such procedures time is of the essence and the use of two or more relaxation curves allows determination of tissue type in less than 5 seconds. But there are also applications where time is less critical. For example, the probe may be used in non-invasive diagnostic procedures where the diagnostician can afford to wait significantly longer, even a minute or more. In such applications, it may not be essential to best-fit the measured response to both curves and use may be made of the T1 characteristic on its own since although it is slower than matching the T2 characteristic it may be sufficient to identify the tissue type while T2 on its own although faster cannot.

### A. T1 measurement with pulse inversion

The purpose of measurement is to measure quantitatively the spin relaxation time T1 of the material. At the beginning of the measurement, before applying the pulses, the system is in a state of thermal equilibrium in which the magnetization is aligned in the direction of the external magnetic field referred to as B0. The first pulse operates to invert the magnetization so that it is aligned in a direction opposite to the magnetic field. As a result, the system strives to return to equilibrium, whereby the time it takes for the system to return to equilibrium is referred to as T1. The purpose of the second and third pulses is to measure the magnetization state at a time t following the first pulse. The measurement is carried out with the aid of an echo signal generated by a combination of 90° and 180° pulses. Repeating this series of pulses with different values of t produces the curve shown in Fig. 2 from which the time constant T1 can be derived.

### B. T2 measurement by a series of 180° pulses

The purpose of this measurement is to measure quantitatively the signal decay time of the magnetic resonance known as T2. This decay is the result of local magnetic fields and spins in the material forming a spread in precession frequencies and causing the signal to decay. This measurement can be made in a single measurement without the need for a series of measurements using the series of pulses shown in Fig. 2c. A suitable algorithm is disclosed by S. Meiboom and D. Gil in "Modified spin-echo method for measuring nuclear relaxation times" Rev. Sci. Instrum. 29, 688 (1958). This series commences with a 90° pulse that shifts the magnetization perpendicular to the direction of the external field. The magnetization now undergoes rotation (precession) around the external field axis. In order not to be subject to inhomogeneity of the external field, use is made of a 180° pulse known as an echo, at the commencement of which frequency spread as the result of a lack of uniformity of the external field is canceled and all the spins are aligned and form a strong signal. Using a series of 180° pulses a series of echoes may be produced thus obtaining from the signal measurement at the start of the echo for the duration of the echo pulses the decay curve as shown in Fig. 2b in which the time constant of the decay is termed T2.

The control unit according to the invention may be a suitably programmed computer. Likewise, the invention contemplates a computer program being readable by a computer for executing the method of the invention. The invention further contemplates a machine-readable memory tangibly embodying a program of instructions executable by the machine for executing the method of the invention.

Features that are described with reference to one or more embodiments are described by way of example rather than by way of limitation to those embodiments. Thus, unless stated otherwise or unless particular combinations are clearly inadmissible, optional features that are described with reference to only some embodiments are assumed to be likewise applicable to all other embodiments also.

## Claims

1. A system (10) for non-invasive analysis of tissue of an examined subject in order to distinguish between different types of tissue including nerves, muscles and blood vessels, the system comprising:
a hand-held probe (15) having a casing (16) formed of non-ferromagnetic material and having a working end (17);
a magnetic field source unit (18) within the casing and being configured to generate a substantially uniform, time-invariant magnetic field within a volume of tissue having a magnetic field strength in a range of 0.02 to 0.75 Tesla within said volume of tissue; and
at least one inductive coil (19) inside the magnetic field source unit and configured to receive RF excitation signals in a specific frequency range related to the magnetic field strength, said at least one inductive coil thereby responding to the magnetic field and to the RF excitation signals by generation of electromagnetic excitation signals in a direction substantially perpendicular to a direction of said magnetic field to thereby magnetize a slice of the living tissue, said slice having a depth and a thickness that are a predetermined function of the magnetic field strength whereby only living tissue in said slice generates an electromagnetic response to nuclear spin echo signals;
a signal generator (20) coupled to the at least one inductive coil and being configured for generating an RF excitation frequency that is a function of the respective electromagnetic response of each slice;
a receiver unit (21) coupled to the at least one inductive coil and being configured to receive therefrom the electromagnetic response and generate measured data indicative thereof; and
a control unit (25) connected to the signal generator so as to generate predetermined time patterns of the excitation RF signals, the control unit being further connected to the receiver unit and responsive to the electromagnetic response for processing the measured data and extracting data indicative of nuclear spin echo signals from the living tissue, to determine relaxation curves having time constants T1 and T2 and statistically process the relaxation curves in order to determine therefrom the type of tissue excited by the probe;
**characterized in that:**
more than one inductive coil is provided and the signal generator (20) is configured to feed to each inductive coil a respective RF excitation signal in mutually different frequency ranges for simultaneously exciting multiple slices at successive depths of body tissue, and
the control unit (25) is responsive to the electromagnetic responses received from said multiple slices for determining relaxation curves for each slice and determining therefrom which type of tissue is excited by the probe in each slice within a group consisting of nerves, muscles and blood vessels.

2. The system according to claim 1, wherein:
the control unit is configured in real time to access predetermined data characterizing multiple groups of relaxation curves,
each group defining either (i) at least one characteristic curve corresponding to a specific known tissue type and decaying exponentially at a respective predetermined time constant T2 or (ii) at least two characteristic curves corresponding to a specific known tissue type and having predetermined time constants T1 and T2, and
the control unit is configured in real time to analyze measured data in real time to determine to which group of relaxation curves the measured data is best fit in order to identify the tissue type corresponding to the measured data.

3. The system according to claim 2, wherein the control unit is configured to augment said predetermined data with time constants and weightings for different exponents determined from the measured data so as to allow successive measurements to be analyzed based on an ever-growing database accumulated with on-going usage and thereby reduce processing time.

4. The system according to claim 3, wherein the control unit is configured to determine to which group of relaxation curves the measured data is best fit in less than 5 seconds from start of measurement.

5. The system according to any one of claims 2 to 4, wherein the probe is calibrated prior to use in order to obtain and store said relaxation curves.

6. The system according to any one of the preceding claims, wherein the working end (17) of the casing (16) has a cylindrical cross-section preferably having a diameter of 2-30 mm.

7. The system according to any one of the preceding claims, wherein the RF excitation frequency lies in a frequency range of 2 to 20 MHz.

8. The system according to any one of claims 1 to 7, wherein at least one of said inductive coils (19) is commonly coupled to the signal generator (20) and to the receiver unit (21) via a duplexer (26) so that the at least one inductive coil applies the RF excitation frequency and receives the response in different time slots.

9. The system according to any one of claims 1 to 7, including multiple inductive coils wherein at least one first inductive coil is coupled to the signal generator and at least one second inductive coil is coupled to the receiver unit, said first and second inductive coils being operative during different time slots for applying the RF excitation frequency and receiving the response.

10. The system according to any one of claims 1 to 9, wherein the magnetic field source unit (18) comprises a pair of outer arcuate segments (30) both of a first magnetic polarity and a pair of inner segments (31) both of a second magnetic polarity opposite to the first magnetic polarity, the at least one inductive coil being disposed within an annular gap (32) between the outer and inner segments.

11. The system according to claim 10, wherein the outer and inner segments have respective contours that lie on circles of different radii.

12. The system according to claim 11, wherein the respective radius of the outer segments is less than 10 mm.

13. The system according to any one of claims 10 to 12, wherein the inner segments (31) are shorter in height than the outer segments (30) and are supported so that respective end faces of the inner and outer segments are co-planar.

14. The system according to any one of the preceding claims, wherein the multiple coils are arranged in a vertical stack with the annular gap between the inner and outer segments.

15. The system according to any one of the preceding claims having multiple probes each directed for exciting a different portion of body tissue.

16. The system according to any one of claims 1 to 13, wherein the at least one inductive coil includes multiple coils each configured to receive a different RF excitation frequency thereby allowing different slices to be analyzed simultaneously.

## Patentansprüche

1. System (10) zur nichtinvasiven Analyse von Körpergewebe eines untersuchten Subjekts zur Unterscheidung von verschiedenen Gewebearte, einschließlich Nerven, Muskeln und Blutgefäßen, wobei das System umfasst:
eine handgeführte Sonde (15), die ein Gehäuse (16) aufweist, das aus einem nicht ferromagnetischen Material hergestellt ist, und die ein Arbeitsende (17) aufweist;
eine Magnetfeldquelleneinheit (18), die sich in dem Gehäuse befindet und dafür konfiguriert ist, ein im Wesentlichen gleichförmiges, zeitinvariantes Magnetfeld in einem Gewebevolumen zu erzeugen, das eine Magnetfeldstärke in einem Bereich von 0,02 bis 0,75 Tesla innerhalb des Gewebevolumens aufweist; und
zumindest eine Induktionsspule (19) innerhalb der Magnetfeldquelleneinheit, die dafür konfiguriert ist, HF-Anregungssignale in einem bestimmten Frequenzbereich bezogen auf die Magnetfeldstärke zu empfangen, wobei die zumindest eine Induktionsspule auf das Magnetfeld und auf die HF-Anregungssignale reagiert, indem sie elektromagnetische Anregungssignale in einer Richtung erzeugt, die im Wesentlichen orthogonal zu einer Richtung des Magnetfelds ist, um somit eine Scheibe des lebenden Gewebes zu magnetisieren, wobei diese Scheibe eine Tiefe und eine Dicke aufweist,
die eine vorbestimmte Funktion der Magnetfeldstärke sind und wobei nur lebendes Gewebe in der Scheibe eine elektromagnetische Antwort auf Kernspin-Echosignale hervorruft;
einen Signalgenerator (20), der mit der zumindest einen Induktionsspule gekoppelt und dafür konfiguriert ist, eine HF-Anregungsfrequenz zu erzeugen, die eine Funktion der entsprechenden elektromagnetischen Antwort jeder Scheibe ist;
eine Empfängereinheit (21), die mit der zumindest einen Induktionsspule gekoppelt und dafür konfiguriert ist, von dieser die elektromagnetische Antwort zu empfangen und diese kennzeichnende Messdaten zu erzeugen; und
eine Steuereinheit (25), die mit dem Signalgenerator verbunden ist, um vorbestimmte Zeitmuster der HF-Anregungssignale zu erzeugen, wobei die Steuereinheit ferner mit der Empfängereinheit verbunden und reaktionsfähig für die elektromagnetische Antwort ist, um die Messdaten zu verarbeiten und Daten zu extrahieren, die kennzeichnend für die Kernspin-Echosignale von dem lebenden Gewebe sind, um Relaxationskurven mit Zeitkonstanten T1 und T2 zu bestimmen, und die Relaxationskurven statistisch zu bearbeiten, um daraus die Art des von der Sonde erregten Gewebes zu bestimmen;
**dadurch gekennzeichnet, dass:**
mehr als eine Induktionsspule bereitgestellt wird und der Signalgenerator (20) dafür konfiguriert ist, jede Induktionsspule mit einem entsprechenden HF-Anregungssignal in voneinander verschiedenen Frequenzbereichen zu speisen, um gleichzeitig mehrere Scheiben in aufeinanderfolgenden Tiefen des Körpergewebes zu erregen, und
die Steuereinheit (25) reaktionsfähig für die elektromagnetischen Antworten ist, die von den mehreren Scheiben empfangen werden, um Relaxationskurven für jede Scheibe zu bestimmen und daraus zu bestimmen, welche Art von Gewebe durch die Sonde in jeder Scheibe erregt wird, innerhalb einer Gruppe bestehend aus Nerven, Muskeln und Blutgefäßen.

2. System nach Anspruch 1, wobei:
die Steuereinheit konfiguriert ist, um in Echtzeit auf vorbestimmte Daten zuzugreifen, die mehrere Gruppen von Relaxationskurven kennzeichnen, jede Gruppe entweder (i) zumindest eine Kennlinie definiert, die einer spezifischen bekannten Gewebeart entspricht und mit einer entsprechenden vorbestimmten Zeitkonstante T2 exponentiell abklingt, oder (ii) zumindest zwei Kennlinien definiert, die einer spezifischen bekannten Gewebeart entsprechen und vorbestimmte Zeitkonstanten T1 und T2 aufweisen, und
die Steuereinheit in Echtzeit dafür konfiguriert ist, die Messdaten in Echtzeit zu analysieren, um zu bestimmen, welcher Gruppe von Relaxationskurven die Messdaten am besten entsprechen, um die Gewebeart zu identifizieren, die den Messdaten entspricht.

3. System nach Anspruch 2, wobei die Steuereinheit dafür konfiguriert ist, die vorbestimmten Daten mit Zeitkonstanten und Gewichtungen für verschiedene Exponenten zu augmentieren, die aus den Messdaten bestimmt werden, um zu ermöglichen, dass nachfolgende Messungen basierend auf einer stetig wachsenden Datenbank, die im fortlaufenden Gebrauch angesammelt wird, analysiert werden können und somit die Verarbeitungszeit reduziert werden kann.

4. System nach Anspruch 3, wobei die Steuereinheit konfiguriert ist, um in weniger als 5 Sekunden ab Messungsbeginn zu bestimmen, welcher Gruppe von Relaxationskurven die Messdaten am besten entsprechen.

5. System nach einem der Ansprüche von 2 bis 4, wobei die Sonde vor dem Gebrauch kalibriert wird, um die genannten Relaxationskurven zu erhalten und zu speichern.

6. System nach einem der vorhergehenden Ansprüche, wobei das Arbeitsende (17) des Gehäuses (16) einen zylindrischen Querschnitt mit einem Durchmesser von vorzugsweise 2-30 mm aufweist.

7. System nach einem der vorhergehenden Ansprüche, wobei die HF-Anregungsfrequenz in einem Frequenzbereich von 2 bis 20 MHz liegt.

8. System nach einem der Ansprüche von 1 bis 7, wobei zumindest eine der Induktionsspulen (19) über einen Duplexer (26) gemeinsam an den Signalgenerator (20) und an die Empfängereinheit (21) gekoppelt ist, so dass die zumindest eine Induktionsspule in verschiedenen Zeitschlitzen die HF-Anregungsfrequenz anlegt und die Antwort empfängt.

9. System nach einem der Ansprüche von 1 bis 7, mehrere Induktionsspulen beinhaltend, wobei zumindest eine erste Induktionsspule an den Signalgenerator gekoppelt ist und zumindest eine zweite Induktionsspule an die Empfängereinheit gekoppelt ist, wobei die ersten und die zweiten Induktionsspulen in unterschiedlichen Zeitschlitzen operativ sind, um die HF-Anregungsfrequenz anzulegen und die Antwort zu empfangen.

10. System nach einem der Ansprüche von 1 bis 9, wobei die Magnetfeldquelleneinheit (18) ein Paar äußerer bogenförmiger Segmente (30), beide mit einer ersten magnetischen Polarität, und ein Paar innerer Segmente (31), beide mit einer zur ersten magnetischen Polarität entgegengesetzten zweiten magnetischen Polarität, umfasst, wobei die zumindest eine Induktionsspule innerhalb eines ringförmigen Spalts (32) zwischen dem äußeren und dem inneren Segment angeordnet ist.

11. System nach Anspruch 10, wobei die äußeren und inneren Segmente entsprechende Konturen aufweisen, die auf Kreisen mit verschiedenen Radien liegen.

12. System nach Anspruch 11, wobei der jeweilige Radius der äußeren Segmente kleiner als 10 mm ist.

13. System nach einem der Ansprüche von 10 bis 12, wobei die inneren Segmente (31) eine kürzere Höhe aufweisen als die äußeren Segmente (30) und so unterstützt werden, dass die jeweiligen Endflächen des inneren und des äußeren Segments koplanar sind.

14. System nach einem der vorhergehenden Ansprüche, wobei die mehreren Spulen in einem vertikalen Stapel mit dem ringförmigen Spalt zwischen dem inneren und dem äußeren Segment angeordnet sind.

15. System nach einem der vorhergehenden Ansprüche, mehrere Sonden aufweisend, die jeweils ausgerichtet sind, um einen verschiedenen Abschnitt von Körpergewebe zu erregen.

16. System nach einem der Ansprüche von 1 bis 13, wobei die zumindest eine Induktionsspule mehrere Spulen beinhaltet, die jeweils dafür konfiguriert sind, eine verschiedene HF-Anregungsfrequenz zu empfangen, um somit das gleichzeitige Analysieren verschiedener Scheiben zu ermöglichen.

## Revendications

1. Un système (10) pour l'analyse non invasive d'un tissu d'un sujet examiné de manière à distinguer différents types de tissus incluant les nerfs, les muscles et les vaisseaux sanguins, le système comprenant :
une sonde à main (15) ayant un boîtier (16) formé de matériau non ferromagnétique et ayant une extrémité de travail (17) ;
une unité de source de champ magnétique (18) à l'intérieur du boîtier et configurée pour générer un champ magnétique essentiellement uniforme, invariable dans le temps, à l'intérieur d'un volume de tissu, ayant une intensité de champ magnétique dans une gamme de 0,02 à 0,75 Tesla à l'intérieur dudit volume de tissu ; et
au moins une bobine inductive (19) à l'intérieur de l'unité de source de champ magnétique et configurée pour recevoir des signaux d'excitation RF dans une gamme de fréquences spécifique liée à l'intensité du champ magnétique, ladite au moins une bobine inductive répondant ainsi au champ magnétique et aux signaux d'excitation RF en générant des signaux d'excitation électromagnétiques dans une direction essentiellement perpendiculaire à une direction dudit champ magnétique pour magnétiser ainsi une tranche du tissu vivant, ladite tranche ayant une profondeur et une épaisseur qui sont une fonction prédéfinie de l'intensité du champ magnétique, de sorte que seul le tissu vivant dans ladite tranche génère une réponse électromagnétique à des signaux d'écho de spin nucléaire ;
un générateur de signaux (20) couplé avec ladite au moins une bobine inductive et configuré pour générer une fréquence d'excitation RF qui est fonction de la réponse électromagnétique respective de chaque tranche ;
une unité réceptrice (21) couplée avec ladite au moins une bobine inductive et configurée pour recevoir de celle-ci la réponse électromagnétique et générer des données mesurées indicatives de celle-ci ; et
une unité de commande (25) reliée au générateur de signaux de manière à générer des modèles temporels prédéfinis des signaux d'excitation RF, l'unité de commande étant en outre reliée à l'unité réceptrice et réagissant à la réponse électromagnétique pour traiter les données mesurées et extraire des données indicatives de signaux d'écho de spin nucléaire provenant du tissu vivant, pour déterminer des courbes de relaxation ayant des constantes de temps T1 et T2 et traiter statistiquement les courbes de relaxation afin de déterminer à partir de celles-ci le type de tissu excité par la sonde ;
**caractérisé en ce que** :
plus d'une bobine inductive est prévu et le générateur de signaux (20) est configuré pour fournir à chaque bobine inductive un signal d'excitation RF respectif dans des gammes de fréquences mutuellement différentes pour exciter simultanément des tranches multiples à des profondeurs successives du tissu corporel, et
l'unité de commande (25) réagit aux réponses électromagnétiques reçues desdites tranches multiples pour déterminer des courbes de relaxation pour chaque tranche et déterminer à partir de celles-ci le type de tissu qui est excité par la sonde dans chaque tranche dans un groupe comprenant les nerfs, les muscles et les vaisseaux sanguins.

2. Le système selon la revendication 1, dans lequel :
l'unité de commande est configurée en temps réel pour accéder à des données prédéfinies caractérisant plusieurs groupes de courbes de relaxation,
chaque groupe définissant soit (i) au moins une courbe caractéristique correspondant à un type de tissu connu spécifique et décroissant exponentiellement à une constante de temps T2 prédéfinie respective soit (ii) au moins deux courbes caractéristiques correspondant à un type de tissu connu spécifique et ayant des constantes de temps T1 et T2 prédéfinies, et
l'unité de commande est configurée en temps réel pour analyser les données mesurées en temps réel afin de déterminer à quel groupe de courbes de relaxation les données mesurées sont le mieux adaptées de manière à identifier le type de tissu correspondant aux données mesurées.

3. Le système selon la revendication 2, dans lequel l'unité de commande est configurée pour augmenter lesdites données prédéfinies avec des constantes de temps et des pondérations pour différents exposants déterminés à partir des données mesurées de manière à permettre aux mesures successives d'être analysées sur la base d'une base de données toujours plus grande accumulée avec une utilisation continue et réduire ainsi le temps de traitement.

4. Le système selon la revendication 3, dans lequel l'unité de commande est configurée pour déterminer à quel groupe de courbes de relaxation les données mesurées sont le mieux adaptées en moins de 5 secondes à compter du début de la mesure.

5. Le système selon l'une quelconque des revendications de 2 à 4, dans lequel la sonde est étalonnée avant l'utilisation de manière à obtenir et à stocker lesdites courbes de relaxation.

6. Le système selon l'une quelconque des revendications précédentes, dans lequel l'extrémité de travail (17) du boîtier (16) a une section transversale cylindrique de préférence ayant un diamètre de 2-30 mm.

7. Le système selon l'une quelconque des revendications précédentes, dans lequel la fréquence d'excitation RF se situe dans une gamme de fréquences de 2 à 20 MHz.

8. Le système selon l'une quelconque des revendications de 1 à 7, dans lequel au moins une desdites bobines inductives (19) est généralement couplée avec le générateur de signaux (20) et avec l'unité réceptrice (21) via un duplexeur (26) de manière à ce que ladite au moins une bobine inductive applique la fréquence d'excitation RF et reçoive la réponse dans des créneaux temporels différents.

9. Le système selon l'une quelconque des revendications de 1 à 7, comprenant plusieurs bobines inductives où au moins une première bobine inductive est couplée avec le générateur de signaux et au moins une deuxième bobine inductive est couplée avec l'unité réceptrice, lesdites première et deuxième bobines inductives étant opérationnelles pendant différents créneaux temporels pour appliquer la fréquence d'excitation RF et recevoir la réponse.

10. Le système selon l'une quelconque des revendications de 1 à 9, dans lequel l'unité de source de champ magnétique (18) comprend une paire de segments arqués extérieurs (30) tous deux d'une première polarité magnétique et une paire de segments intérieurs (31) tous deux d'une deuxième polarité magnétique opposée à la première polarité magnétique, ladite au moins une bobine inductive étant disposée dans un espace annulaire (32) entre les segments extérieurs et intérieurs.

11. Le système selon la revendication 10, dans lequel les segments extérieurs et intérieurs ont des contours respectifs qui se situent sur des cercles de différents rayons.

12. Le système selon la revendication 11, dans lequel le rayon respectif des segments extérieurs est inférieur à 10 mm.

13. Le système selon l'une quelconque des revendications de 10 à 12, dans lequel les segments intérieurs (31) sont plus courts en hauteur que les segments extérieurs (30) et sont supportés de manière à ce que des faces d'extrémité respectives des segments intérieurs et extérieurs soient coplanaires.

14. Le système selon l'une quelconque des revendications précédentes, dans lequel les bobines multiples sont disposées en une pile verticale dans l'espace annulaire entre les segments intérieurs et extérieurs.

15. Le système selon l'une quelconque des revendications précédentes, ayant plusieurs sondes dirigées chacune pour exciter une portion différente de tissu corporel.

16. Le système selon l'une quelconque des revendications de 1 à 13, dans lequel ladite au moins une bobine inductive comprend des bobines multiples configurées chacune pour recevoir une fréquence d'excitation RF différente, permettant ainsi l'analyse simultanée de différentes tranches.
